# EUROPEAN PATENT SPECIFICATION

(11) **EP 3 217 885 B1**
(45) Date of publication and mention of the grant of the patent: **13.05.2020**
(21) Application number: 15808800.5
(22) Date of filing: 10.11.2015
(51) Int. Cl.: A61B 8/00

(54) **HANDHELD ULTRASOUND SCANNER**
HANDHALTBARER ULTRASCHALLSCANNER
DISPOSITIF DE BALAYAGE À ULTRASONS PORTATIF

(30) Priority: 13.11.2014 IT MI20141962
(43) Date of publication of application: 20.09.2017
(73) Proprietor: Innuvatech S.r.l., 20133 Milan (IT)
(72) Inventor: STRAMIGIOLI, Ludovico, 16100 Genova (IT)
(74) Representative: Lunati & Mazzoni S.r.L.
(86) International application number: PCT/IB2015/058670
(87) International publication number: WO 2016/075618

(56) References cited:
- WO-A1-2014/041448
- US-A1- 2003 097 071
- US-B1- 7 549 961

## Description

The present invention relates to an operating procedure of a handheld ultrasound scanner and an ultrasound scanning apparatus according to the claims.

Ultrasound scanners for diagnostic, hospital or other purposes are currently known of.

An example of an ultrasound scanner of such type is described in the patent application US-A-2014/041448.

These devices make it possible to obtain images of the inside of the human body, in particular soft tissue.

They work by generating and sending ultrasound pulses from the outside to the inside of the human body. Said ultrasound pulses rebound, in an echo effect, against the inner tissues and are received back by the ultrasound scanner distorted or changed after the echo.

The ultrasound scanner picks up the modified ultrasound pulses and turns them into electromagnetic waves by means of specific transducers. The electromagnetic waves, properly interpreted, thus contain information about the surfaces inside the human body which created the echo effect.

The electromagnetic waves are then transformed into still or moving images by specific processors. These images show the inside of the human body. Structurally, the ultrasound scanners comprise a machine body wired to a probe. The probe comprises said transducers and other servo elements, while the machine body generally comprises a processing and calculation centre, the means for controlling the generation of ultrasound pulses, the electromagnetic wave receiving means, the screen and all the necessary devices. In addition, each ultrasound scanner preferably comprises a plurality of interchangeable probes.

In particular two different kinds of ultrasound scanners are made: fixed and handheld ultrasound scanners.

The fixed systems are large and highly precise and are generally placed inside hospitals or outpatient clinics in dedicated areas.

The handheld ultrasound machines are less precise and smaller. They comprise a machine body in most cases, the size of a briefcase.

Ultrasound scanners have several important advantages. In particular they have no significant side effects, do not radiate the human body and are sufficiently precise.

The prior art mentioned above has several significant drawbacks.

In fact, conventional ultrasound scanners are cumbersome.

Even handheld ultrasound scanners, despite the small size, are still very expensive.

Such drawbacks particularly affect home visits, and small medical or veterinary practices.

In this situation the technical purpose of the present invention is to devise a handheld ultrasound scanner able to substantially overcome the drawbacks mentioned above.

Within the sphere of said technical purpose one important aim of the invention is to devise an economical and sufficiently accurate handheld ultrasound scanner. Another important aim of the invention is to make a compact handheld ultrasound scanner.

The technical purpose and specified aims are achieved by an operating procedure of a handheld ultrasound scanner and an ultrasound scanning apparatus according to the appended claims.

Preferred embodiments are evident from the dependent claims.

The characteristics and advantages of the invention are clearly evident from the following detailed description of a preferred embodiment thereof, with reference to the accompanying drawings, in which:

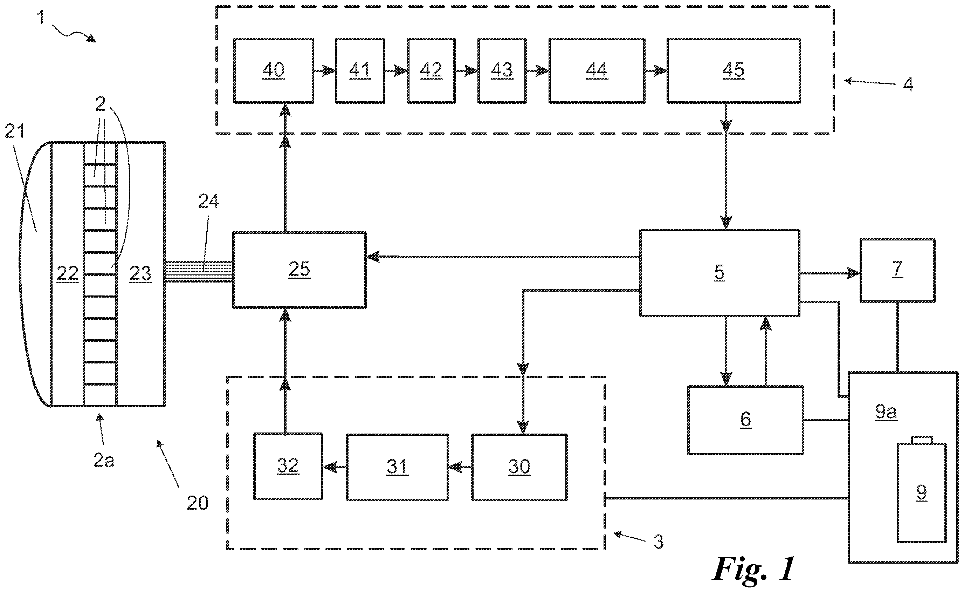

**Fig. 1** shows an operating diagram of the handheld ultrasound scanner according to the invention;

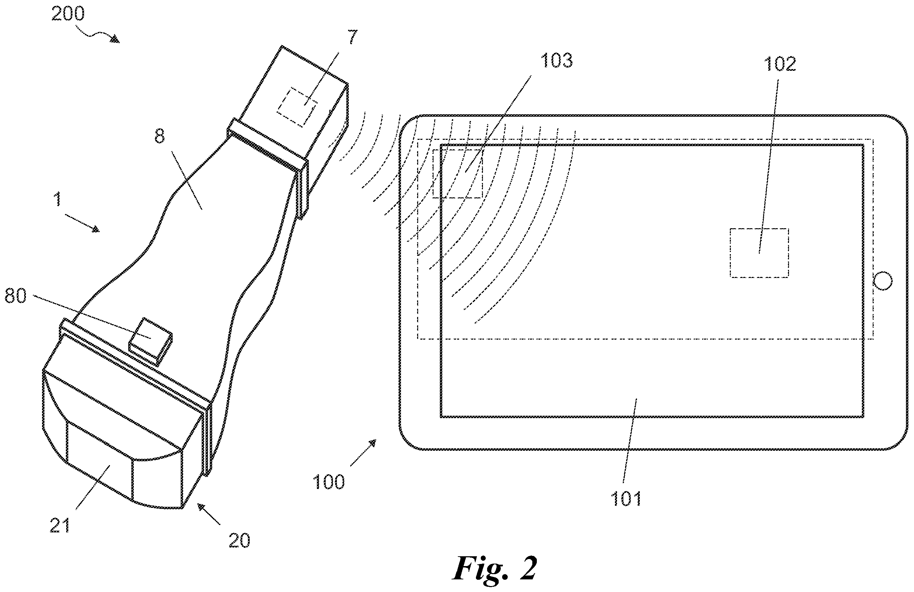

**Fig. 2** shows the handheld ultrasound scanner according to the invention used together with a conventional device.

With reference to said drawings, reference numeral **1** globally denotes the handheld ultrasound scanner according to the invention.

It is suitable for performing ultrasound scans on bodies, particularly on human bodies, animals or structures of various types.

The handheld ultrasound scanner 1 is associated with an external computer **100** of the conventional type for general purposes with sundry software and a generally-used and versatile operating system. The computer 100 preferably consists of a smartphone or tablet, for example a smartphone or tablet running an Android, IOS®®, Windows®, Linux or similar operating system.

The computer 100 comprises a display screen **101** electronic processing means **102** such as a processor or the like, preferably of the ARM® Cortex® or Intel® Atom® type or more powerful. It further comprises connections **103**, such as cable input jacks (USB, Apple Lightning connector ® or HDMI, Thunderbolt® or so on) and in particular wireless connections, i.e. receiving and transmitting antennas, for example Bluetooth® or Wi-Fi®.

The handheld ultrasound scanner 1 and the external computer 100 form an ultrasound apparatus **200** which comprises them both.

The handheld ultrasound scanner 1 comprises transducers **2** suitable to convert electromagnetic pulses into ultrasound pulses and vice versa and, thus, to send and receive ultrasound pulses.

The transducers 2 are known per se and preferably over 16 in number, more preferably 32 or 64 in number and appropriately in a phased array configuration, known per se to a person skilled in the art. The set of transducers 2 is called a transducer array **2a.**

The transducers 2 are part of a head **20** which comprises, in addition to said transducers 2, an acoustic lens **21**, suitable to minimise the acoustic impedance of the interface between the body to be examined and the handheld ultrasound scanner 1, and constituting the proximal end of the ultrasound scanner 1. The acoustic lens 21 and transducers are connected by a coupling material **22** suitable to mitigate the impedance of the lens 21. Distal to the transducers 2 is a damping material **23** to prevent the acoustic waves from moving up the probe.

The head 20 is further, immediately connected by an analogue electric connector **24** preferably quickly detachable and per se known, to a switch **25** preferably of the T/R type, namely both transmitting and receiving. It is a high impedance switch 25 allowing communication between the transducer array 2a and the remaining part of the ultrasound scanner 1 in receiving or transmitting, without the latter interfering with each other.

The handheld ultrasound scanner 1 then comprises electromagnetic wave generating means **3** suitable to send given electromagnetic pulses, i.e. of the chosen type to transducers 2 to convert them into given ultrasound pulses, i.e. ultrasound pulses of the chosen frequency and duration. They are then connected to the transducers 2, and then to the head 20, in particular to the switch 25 and consequently to the head 20.

The electromagnetic wave generating means 3 comprise a transmission beamformer **30**, of the transmissive type, suitable to create digital pulses and to send them to a pulse generator **31** of another voltage (HV pulsar), also part of said electromagnetic wave generating means 3, suitable to convert said digital pulses into analogue pulses. The analogue pulse created by the pulse generator 31 is sent to a transmission de-muxer **32** or demultiplexer, part of said generating means 3, which divides the analogue signal into a plurality of signals and sends them to individual transducers 2 part of the array 2a. Said components are known per se.

The handheld ultrasound scanner 1 further comprises electromagnetic wave receiving means **4** suitable to receive electromagnetic pulses from the transducers 2 consequent to the reception of ultrasound echoes of the ultrasound pulses sent, and therefore following their echo on the body in question. They are in addition suitable to convert the analogue signal coming from the transducers 2 into a digital signal, and to offset the effects of phase shift and attenuation which the signal undergoes during the transit in the body tissues examined.

The receiving means 4 are then connected to the head 20 and in particular by means of the switch 25 and receive the signals in output from the same.

The receiving means 4 may initially comprise a receiving muxer **40** suitable to gather the analogue signals coming from the plurality of transducers 2 into one signal.

The receiving means 4 then comprise, preferably immediately downstream of the receiving muxer 40, an LNA **41** or low noise amplifier, suitable to amplify the weak signals coming from the array 2a.

The receiving means 4 then comprise, preferably after the LNA 41, a Time Gain Compensation TGC **42.** This has the function of amplifying signals in inverse proportion to the acquisition time thereof, i.e. in proportion to the depth of penetration. The TGC 42, known per se, thus has the function of offsetting the attenuation of the ultrasound pulses coming from distal structures occurring in the body tissue examined.

Downstream of the TGC 42, the receiving means 4 appropriately comprise a converter **43** suitable to transmute the analogue signal into digital and per se known.

The converter 43 is preferably followed by a receiving de-muxer **44** which again divides the signal, previously consisting of a plurality of signals received from different transducers 2 then assembled by the receiving muxer 40, into a plurality of respective signals, preferably digital.

Downstream of the receiving de-muxer 44 is a receiving beamformer **45**, known per se. It is an electronic element, sometimes integrated with the TGC, which puts the incoming signals back into phase and sums them to then possibly eventually demodulate them in quadrature phase.

The handheld ultrasound scanner 1 further comprises control means **5** suitable to control the components of said handheld ultrasound scanner 1. Said control means 5 are preferably directly connected to the switch 25, and then to the head 20, the receiving means 4, the generating means 3 and to other elements described below. In particular, the control means 5 are connected to the receiving 45 and transmission beamformers 30.

The control means 5 are in particular suitable to receive signals from the receiving means 4 and in particular from the receiving beamformer 45 and to send signals to the generating means 3 and in particular to the transmission beamformer 30. They are suitable in particular to control the activation or deactivation of the generating means 3 and receiving means 4 and to control the direction in input or output of the signal from or to the transducers 2 by means of the switch 25.

The control means 5 comprise a microprocessor, for example an ARM® processor or the like, and may further comprise a DSP (digital signal processor), and/or an FPGA or in any case a microcontroller.

The control means 5 are connected, in transmission and reception to processing means **6** suitable to process the signals coming from the control means 5 and preferably not substantially modified thereby. The processing means 6 thus receive the signal coming from the receiving means 4 and apply thereto known algorithms, such as, preferably, the B-Mode method or even the color doppler or otherwise, for the reconstruction of the ultrasound scan image.

Structurally the processing means 6 may consist of an ARM® processor and/or of a multicore DSP or similar.

After obtaining the image the processing means 6 send it back to the control means which possibly perform a brief check and send it to the transmitting **7** means, part of the handheld ultrasound scanner 1.

The transmitting means 7 are preferably of the wireless type and are thus composed at least of a transmitting antenna. More preferably, the transmitting means 7 are a Bluetooth® module, in particular type 2.0 or subsequent, or alternatively a Wi-Fi module or otherwise. Alternatively the transmitting means are of the wired type, in particular of the Apple Lightning connector USB® or HDMI® type or otherwise.

The handheld ultrasound scanner 1 comprises then a battery **9** and management means thereof, suitable to optimise its charging. The battery 9 is connected at least to the control means 5, the generating means 3, the transmitting means 7 and to all the elements requiring electricity.

Structurally, the handheld ultrasound scanner 1 comprises a containment casing **8**, preferably rigid and preferably made of polymeric material. It is suitable to connect mechanically, and preferably integrally with each other, the elements described above, which make up the handheld ultrasound scanner 1, in particular: the generating means 3, the receiving means 4, the control means 5, the processing means 6, the transmitting means 7, the battery 9 and connected circuit board and the head 20. Preferably the casing 8 comprises solely said elements.

In more detail the head 20 and in particular the acoustic lens 21, forms the distal end of the handheld ultrasound scanner 1. It is additionally preferably integrally constrained to the casing 8 by means of releasable constraint means, for example by a join between the two elements, so that said head may be simply replaced and is interchangeable. For the purposes of interchangeability, the head 20 preferably also comprises an electric connector 24 with the switch 25 of the detachable type, or a detachable electrical connection between the switch 25 and the remaining part of the ultrasound scanner 1.

The casing 8 further comprises an activation button **80** and connection means for recharging the battery 9 or a connection to replace the same.

The functioning of the handheld ultrasound scanner 1, described above in structural terms, is as follows. Said functioning defines an innovative new operating procedure of a handheld ultrasound scanner 1, which is implemented precisely by means of the handheld ultrasound scanner 1 described above.

In said procedure the handheld ultrasound scanner 1, in particular the acoustic lens 21, is placed in contact with the human body or animal to be analysed. Alternatively, the handheld ultrasound scanner 1 is placed on the object to be examined.

The handheld ultrasound scanner 1 is activated using the button 80.

The control means 5 send the generating means 3 the command to activate and generate electromagnetic waves and at the same time set the switch 25 so that it lets the signals pass from the generating means 3 to the head 20 and not vice versa. Consequently, the transmission beamformer 30 creates the digital impulses and sends them to the pulse generator 31 which converts them into analogue impulses or waves. The analogue impulses are sent to the transmission de-muxer 32 which divides them into a plurality of signals and sends them to the head 20 and then to individual transducers 2, part of the array 2a, by means of the switch 25 properly set, to the connector 24 and the coupling material 23.

The electromagnetic waves are transformed into vibrations by the transducers 2. Said vibrations have a frequency such as to create ultrasound pulses which pass through the acoustic lens 21, enter the body to be examined and return, through an echo effect, to said acoustic lens 21 and from there to the transducers 2.

At this point, and at preselected and regular time intervals, the control means 5 set the switch 25 so that it lets the signals pass from the head 20 to the receiving means 4 and not otherwise.

The transducers 2 convert the vibrations or ultrasound pulses received into electromagnetic signals and send them, through the coupling material 23 and the switch 25, properly set up, to the receiving means 4.

In particular the signals from the various transducers 2 reach the receiving muxer 40 and are grouped into a smaller number of channels. The signal arrives then at the LNA 41, suitable to amplify weak signals coming from the array 2a, and from here to the TGC 42 which amplifies the signals in inverse proportion to the acquisition time thereof, offsetting the attenuation of the ultrasound pulses coming from distal structures which occurs in the tissue of the body examined.

The signal arrives then at the converter 43, which converts it into digital and at the receiving de-muxer 44, which re-divides the signal into a plurality of respective signals, preferably digital, coming from the individual arrays.

The signal then reaches the receiving beamformer 45, which puts the incoming signals back into phase, sums them, and possibly demodulates them in a quadrature phase, and from here back to the control means 5.

To sum up, principally, the receiving means 4 receive electromagnetic impulses from the head 20 and from the transducers 2, resulting from the receipt of the echo ultrasound pulses of the ultrasound pulses sent and convert the analogue signal into a digital signal, offsetting the effects of phase shift and attenuation which the signal undergoes in particular during the transit in the body tissues examined. The control means 5 in turn send the signal to the processing means 6 which process the received signal, in a manner known per se, to obtain still or moving images. In particular and advantageously the processing means 6 apply to incoming signal the standard algorithm for the reconstruction of the ultrasound image. They thus preferably obtain a series of black and white and circular sector images. Preferably 30 images per second are obtained.

Said data are sent back to the control means 5, which checks the correctness thereof, and from there to the transmitting means 7 which transmit them externally. In particular, the transmitting means 7 are advantageously wireless, for example Bluetooth®, in particular type 2.0 or subsequent, or alternatively Wi-Fi® and are suitable to transfer in a wireless manner said amount of data and said images so as not to require cables or the like. In particular, it is appropriate that said wireless transmitting means 7 have a transmitting/receiving speed greater than or equal to 3Mbit/s. Said transmitting means 7 permit a universal interface with the computers 100, already generally provided with said interfaces.

The data processed by the processing means 6 are then sent to the computer 100, in particular the connection 103, of the same type as the transmitting means 7, thus wired or preferably wireless, more preferably Bluetooth® in particular type 2.0 or subsequent, or alternatively Wi-Fi, preferably with a receiving/transmitting speed greater than or equal to 3Mbit/s.

The computer 100 appropriately comprises, in its memory, a software suitable to complete the process, by means of electronic processing means 102, which consist of various types of processors.

The computer 100 then performs a further processing of the data and transforms the series of circular sector images to rectangular passing from polar coordinates to Cartesian coordinates, and optionally interpolates the images for added definition.

The computer 100 then creates moving images 100 which appear on the screen 101 of said computer 100. The viewing of the moving images thus entails further possible data processing related to the viewing, such as the possible unpacking of compressed data or the mere transfer of information.

The computer 100 in particular performs operations of so-called post-processing of the data, in particular operations known as Steckle, or other operations.

The data processing procedure is thus divided between the ultrasound scanner 1 and the computer 100 and divided appropriately so as to allow to both the handheld ultrasound scanner 1 and the computer 100 to handle the processing speed of the received signals to turn them into moving images and to allow the wireless transmitting means 7 to transmit said data.

The split point is that described, in which the processing means 6, part of the ultrasound scanner 1, perform the standard algorithm for image reconstruction from the ultrasound signal coming from the receiving means 4, preferably creating instead a series of circular sector images in black and white, preferably 30 images per second, while the processor 102 part of the computer 100 runs the rest of the process to create the video file and display it.

The computer 100 lastly comprises controls for the ultrasound scanner, for example of the touch-screen type to take snapshots of the moving images, to take measurements, to highlight details and so on.

The invention achieves some important advantages.

In fact, the handheld ultrasound scanner 1 is extremely compact and small in volume.

This is due to the fact that the probe is integrated in the ultrasound scanner in one piece and also by the fact that, to see the results, and preferably also to partially process the same, a computer 100, such as a tablet or smartphone, is used which the doctor normally brings with him.

The handheld ultrasound scanner 1 it also very economical, mostly for the same reasons that make it compact.

Lastly, the handheld ultrasound scanner 1 has a universal communication interface compatible with computers 100 known per se and used.

Variations may be made to the invention without departing from the scope of the inventive concept described in the claims.

## Claims

1. Operating procedure of a handheld ultrasound scanner (1),
said handheld ultrasound scanner (1) comprising:
- transducers (2) suitable to convert electromagnetic pulses into ultrasound pulses and vice versa and, thus, to send and receive ultrasound pulses,
- electromagnetic wave generating means (3) suitable to send given electromagnetic pulses to said transducers (2) to be converted into given ultrasound pulses,
- electromagnetic wave receiving means (4) suitable to receive electromagnetic pulses from said transducers (2) consequent to the reception of ultrasound echoes of the ultrasound pulses sent,
- control means (5) suitable to control the components of said handheld ultrasound scanner (1),
- processing means (6) suitable to process signals from said receiving means (4) and convert them into images,
and wherein said receiving means (4) receive electromagnetic pulses from said transducers (2) and at least convert the analogue signal into a digital signal, and offset any effects of phase shift or attenuation of the signal received,
- wherein
- said handheld ultrasound scanner (1) comprises transmitting means (7) for transmitting said images to a computer (100) of a conventional type external to said handheld ultrasound scanner (1), preferably consisting of a smartphone or tablet, and said computer (100) comprising a screen (101), electronic processing means (102), connections (103), controls for said ultrasound scanner, preferably of the touch-screen type,
- wherein said procedure further consists of:
- processing said signals coming from said receiving means (4) by means of said processing means (6) contained in said ultrasound scanner, and converting them into images by means of a B-Mode processing,
- sending said images processed by said processing means (6) to said computer (100) by means of said transmitting means(7),
- said procedure being **characterised in that**
- the processing means (6) reconstructs said signals into a series of black and white circular sector images, while processor (102) performs operations of post-processing of said images which results in the creation of a video file,
- the viewing of said video by said computer (100) on said screen (101).

2. Procedure as claimed in the previous claim, wherein said handheld ultrasound scanner (1) comprises a containment casing (8), and wherein said transducers (2), said electromagnetic wave generating means (3), said receiving means (4), said control means (5) and at least part of said processing means (6) and of said transmitting means (7) are integrally constrained to said casing (8).

3. Ultrasound scanning apparatus (200) comprising a handheld ultrasound scanner (1) and a computer of conventional type (100), the apparatus (200) implementing the procedure according to one of the previous claims.

4. Ultrasound scanning apparatus according to the previous claims, wherein said transmitting means (7) is of the wireless type.

5. Ultrasound scanning apparatus (200) according to claim 3 or 4,
wherein said computer (100) is a tablet or smartphone of the type normally used for general purposes with sundry software and a generally-used and versatile operating system.

## Patentansprüche

1. Betriebsverfahren eines handhaltbaren Ultraschallscanners (1),
wobei der genannte handhaltbare Ultraschallscanner (1) Folgendes umfasst:
- Wandler (2), die geeignet sind, elektromagnetische Impulse in Ultraschall zu verwandeln und umgekehrt und folglich Ultraschall zu versenden und zu empfangen,
- Elemente zum Erzeugen (3) von elektromagnetischen Wellen, die geeignet sind, bestimmte elektromagnetische Impulse an die genannten Wandler (2) zu senden, um diese in bestimmten Ultraschall umzuwandeln,
- Elemente zum Empfangen (4) von elektromagnetischen Wellen, die geeignet sind, elektromagnetische Impulse von den genannten Wandlern (2) zu empfangen, nachdem Echo-Ultraschall des versandten Ultraschalls empfangen wurde,
- Steuerelemente (5), die geeignet sind, die Komponenten des handhaltbaren Ultraschallscanners (1) zu steuern,
- Verarbeitungselemente (6), die geeignet sind, von den genannten Elementen zum Empfangen (4) kommende Signale zu verarbeiten und in Bilder umzuwandeln,
und bei dem die genannten Elemente zum Empfangen (4) elektromagnetische Impulse von den genannten Wandlern (2) erhalten und zumindest das analoge Signal in ein digitales Signal verwandeln und die Wirkungen der Phasenverschiebung und Abschwächung des erhaltenen Signals ausgleichen,
- bei dem: der genannte handhaltbare Ultraschallscanner (1) Elemente zum Übertragen (7) der genannten Bilder an einen Computer (100) bekannten Typs außerhalb des genannten handhaltbaren Ultraschallscanners (1) umfasst, vorzugsweise in Form eines Smartphones oder Tablets, wobei der genannte Computer (100) einen Bildschirm (101), elektronische Verarbeitungsmittel (102), Anschlüsse (103), Steuerungen für den genannten Ultraschallscanner, vorzugsweise Steuerungen über Touchscreen, umfasst
- und bei dem das genannten Verfahren außerdem in Folgendem besteht:
- der Verarbeitung der über die genannten Verarbeitungselemente (6) im Inneren des genannten Ultraschallscanners von den genannten Elementen zum Empfangen (4) kommenden Signalen und deren Umwandlung in Bilder über ein B-Mode-Verfahren,
- dem Versand der von den genannten Verarbeitungselementen (6) verarbeiteten genannten Bilder oder Aufnahmen an den genannten Computer (100) über die genannten Übertragungselemente (7),
wobei das genannte Verfahren **dadurch gekennzeichnet ist, dass**:
- die genannten Verarbeitungselemente (6) das genannte Signal in einer Reihe von Schwarz-Weiß-Bildern mit Kreissektor rekonstruieren, während der Prozessor (102) Vorgänge der Nachbearbeitung dieser Bilder durchführt, die die Erstellung einer Videodatei und
- das Einblenden des genannten Videos von Seiten des genannten Computers (100) auf dem genannten Bildschirm (101) gestatten.

2. Verfahren nach dem vorangegangenen Anspruch, bei dem der genannte handhaltbare Ultraschallscanner (1) ein Gehäuse (8) umfasst und bei dem die genannten Wandler (2), die genannten Elemente zum Erzeugen (3) von elektromagnetischen Wellen, die genannten Elemente zum Empfangen (4), die genannten Steuerelemente (5) und mindestens ein Teil der genannten Verarbeitungselemente (6) und der genannten Übertragungselemente (7) fest mit dem genannten Gehäuse (8) verbunden sind.

3. Ultraschallgerät (200), umfassend einen handhaltbaren Ultraschallscanner (1) und einen Computer (100) bekannten Typs, wobei das Gerät das Verfahren nach einem oder mehreren der vorangegangenen Ansprüche implementiert.

4. Ultraschallgerät (200) nach dem vorangegangenen Anspruch, bei dem die genannten Übertragungselemente (7) schnurlosen Typs sind.

5. Ultraschallgerät (200) nach Anspruch 3 oder 4, bei dem der genannten Computer (100) ein üblicherweise zu allgemeinen Zwecken mit Software unterschiedlichen Typs und allgemeinem und vielseitigem Betriebssystem verwendetes Tablet oder Smartphone ist.

## Revendications

1. Procédure de fonctionnement d'un dispositif de balayage à ultrasons portatif (1),
ledit dispositif de balayage à ultrasons portatif (1) comprenant :
- des transducteurs (2) permettant de transformer des impulsions électromagnétiques en ultrasons et vice versa, et donc d'envoyer et de recevoir des ultrasons,
- des moyens de génération (3) d'ondes électromagnétiques permettant d'envoyer des impulsions électromagnétiques déterminées auxdits transducteurs (2) pour les transformer en ultrasons déterminés,
- des moyens de réception (4) d'ondes électromagnétiques permettant de recevoir des impulsions électromagnétiques desdits transducteurs (2) après réception d'ultrasons écho des ultrasons envoyés,
- des moyens de contrôle (5) permettant de commander les composants dudit dispositif de balayage à ultrasons portatif (1),
- des moyens d'élaboration (6) permettant d'élaborer des signaux provenant desdits moyens de réception (4) et de les transformer en images,
et dans laquelle lesdits moyens de réception (4) reçoivent des impulsions électromagnétiques desdits transducteurs (2) et au moins transforment le signal analogique en signal numérique tout en compensant les effets de décalage et d'atténuation du signal reçu,
- où : ledit dispositif de balayage à ultrasons portatif (1) comprend des moyens de transmission (7) desdites images sur un ordinateur (100) de type connu, externe par rapport audit dispositif de balayage à ultrasons portatif (1), préférablement constitué par un smartphone ou une tablette, et ledit ordinateur (100) comprenant un écran (101), des moyens électroniques d'élaboration (102), des connexions (103), des commandes pour ledit dispositif de balayage à ultrasons, préférablement de type touch-screen,
- et où ladite procédure consiste en outre à :
- l'élaboration desdits signaux provenant desdits moyens de réception (4) par lesdits moyens d'élaboration (6) internes dudit dispositif de balayage à ultrasons, et transformation en image selon la méthode B-Mode,
- l'envoi desdites images ou films élaborés par lesdits moyens d'élaboration (6) sur ledit ordinateur (100) par l'intermédiaire desdits moyens de transmission (7),
cette procédure étant **caractérisée en ce que** :
- lesdits moyens d'élaboration (6) reconstruisent ledit signal en une série d'images à secteur circulaire en noir et blanc, tandis que le processeur (102) réalise des opérations de post-édition desdites images qui permet la création d'un fichier vidéo,
- l'affichage de cette vidéo sur ledit écran (101) dudit ordinateur (100).

2. Procédure selon la revendication précédente, dans laquelle ledit dispositif de balayage à ultrasons portatif (1) comprend une coque de protection (8), et dans laquelle lesdits transducteurs (2), lesdits moyens de génération (3) d'ondes électromagnétiques, lesdits moyens de réception (4), lesdits moyens de contrôle (5) et au moins une partie desdits moyens d'élaboration (6) et desdits moyens de transmission (7) sont solidairement rattachés à ladite coque (8).

3. Système de balayage (200) comprenant un dispositif de balayage à ultrasons portatif (1) et un ordinateur (100) de type connu, le système mettant en œuvre la procédure selon l'une ou plusieurs des revendications précédentes.

4. Système de balayage à ultrasons (200) selon la revendication précédente, dans lequel lesdits moyens de transmission (7) sont de type sans fil.

5. Système de balayage à ultrasons (200) selon la revendication 3 ou 4, dans lequel ledit ordinateur (100) est une tablette ou un smartphone couramment utilisé(e) à des fins générales avec divers types de logiciels et un système d'exploitation commun et polyvalent.
